# EUROPEAN PATENT SPECIFICATION

(11) **EP 3 893 957 B1**
(45) Date of publication and mention of the grant of the patent: **16.10.2024**
(21) Application number: 19813559.2
(22) Date of filing: 06.12.2019
(51) Int. Cl.: A61M 60/13, A61M 60/174, A61M 60/216, A61M 60/857

(54) **CATHETER FOR INTRAVASCULAR BLOOD PUMP**
KATHETER FÜR INTRAVASKULÄRE BLUTPUMPE
CATHÉTER POUR POMPE À SANG INTRAVASCULAIRE

(30) Priority: 11.12.2018 EP 18211647
(43) Date of publication of application: 20.10.2021
(73) Proprietor: Abiomed Europe GmbH, 52074 Aachen (DE)
(72) Inventor: MOURRAN, Claudia, 52074 Aachen (DE); SIESS, Thorsten, 52074 Aachen (DE)
(74) Representative: Klunker IP Patentanwälte PartG mbB
(86) International application number: PCT/EP2019/084092
(87) International publication number: WO 2020/120342

(56) References cited:
- WO-A1-2012/034056
- WO-A1-2012/129383
- WO-A1-2013/062773
- WO-A1-2013/176746
- WO-A1-2018/089970
- WO-A2-2005/120622
- US-A1- 2003 083 539

## Description

### FIELD OF THE INVENTION

This invention relates to an intravascular blood pump for percutaneous insertion into a patient's blood vessel, in particular to be advanced into the patient's heart, and more specifically to a catheter of the intravascular blood pump which reduces or prevents ingrowth into tissue of the blood vessel.

### BACKGROUND OF THE INVENTION

An intravascular blood pump, which is designed to be inserted percutaneously into a patient's blood vessel, such as a femoral or axillary artery or vein, may be advanced into the patient's heart to act as a left ventricular assist device or right ventricular assist device. The blood pump may, thus, be referred to also as an intracardiac blood pump. An intravascular blood pump typically comprises a catheter and a pumping device attached to a distal end of the catheter. The catheter may have an elongate tubular body and may contain supply lines, such as an electric line and a purge line. The pumping device may comprise an impeller which rotates during operation of the blood pump to convey blood from a blood flow inlet to a blood flow outlet of the blood pump, for instance through a flow cannula. Throughout this disclosure, the term "distal" will refer to directions away from a user and towards the heart, whereas the term "proximal" will refer to directions towards a user.

Intravascular blood pumps may be applied in short-term applications for a few hours or days or in long-term applications for weeks or even months. Typically, an intravascular blood pump may be used as a bridge to recovery, which means that the blood pump is removed from the patient after the heart has reached a sufficient recovery and no longer needs to be supported by the blood pump. However, particularly in long-term applications, removal of the blood pump may be difficult because it may be encapsulated by tissue of the blood vessel. Encapsulation of foreign objects by tissue overgrowth and possibly ingrowth is a natural reaction of the human's body against foreign objects to form a protection barrier between the body and the foreign object inside the body. Generally, the material and surface of the foreign object, its geometry, mechanical irritation caused by the object and the like trigger tissue encapsulation of the foreign object.

With respect to an intravascular blood pump, particularly the catheter, which extends through a blood vessel towards the heart, may be treated as a foreign object by the patient's body and may thus be subject to tissue ingrowth or overgrowth due to its proximity to the vessel wall. In particular, mechanical irritations of the inner vessel wall caused by contact with the catheter may promote tissue ingrowth. Tissue ingrowth is a relevant problem in long-term applications, for instance 30 days or longer operation of the blood pump. High forces (e.g. up to 50 N) may be necessary for removal of the blood pump, which would cause severe trauma and injuries to the blood vessel and can lead to particulate emissions, which in turn may cause infarcts.

Different attempts have been made to reduce or avoid ingrowth of the catheter in the blood vessel. However, tests are difficult because apparently different species react differently with regards to tissue ingrowth or overgrowth. Tissue ingrowth or overgrowth starts on the interior of the vessel lumen and on the implant side with adsorption of fibrinogen on the outer surface of the catheter. After that, platelets (thrombocytes) and red blood cells (RBCs, erythrocytes) adhere to the fibrinogen layer. Finally, a thin sleeve containing fibrin forms on the outer surface of the catheter.

In order to avoid creation of such sleeve, catheters usually are provided with smooth surfaces, e.g. formed of extruded polyurethane (PU). However, creation of such sleeve will still take place, which may adhere to the vessel wall or may crumple on the smooth polyurethane surface. If the sleeve loosens, it may at least partially tear off such that an infarct may be caused. In order to avoid adsorption of fibrinogen, organic or inorganic (e.g. Ag) additives may be added to the polyurethane. Likewise, antithrombogenic, hydrophilic or hydrophobic coatings may be applied. Nevertheless, tissue ingrowth is still a problem and is a critical barrier for long-term applications for intravascular blood pumps.
WO 2012/034056 A1 discloses an indwelling medical device which is capable of delivering a therapeutic agent evenly along the length of the indwelling portion, including the outer wall, of the device.

### SUMMARY OF THE INVENTION

It is therefore an object of the present invention to provide an intravascular blood pump, more specifically a catheter for an intravascular blood pump, which allows easy removal of the blood pump from the patient after a certain amount of time, in particular in long-term applications, by reducing or avoiding tissue ingrowth.

This object is achieved according to the present invention by a catheter having the features of claim 1. Preferred embodiments and further developments of the invention are specified in the claims dependent thereon.

According to an aspect of the invention, a catheter for an intravascular blood pump for percutaneous insertion into a patient's blood vessel is provided. According to another aspect, an intravascular blood pump for percutaneous insertion into a patient's blood vessel is provided which comprises a pumping device and the catheter. The catheter of both aspects has an elongate tubular body which extends between a proximal end and a distal end and has an outer surface. According to the invention, at least a portion of an outer surface of the catheter is configured to promote adsorption of proteins, in particular blood proteins, most preferably fibrinogen.

In one embodiment, the catheter includes a porous three-dimensional structure on at least a portion of the outer surface. The structure is porous, i.e. it has a plurality of apertures, which allow adsorption of blood proteins and other cells. The structure is three-dimensional, i.e. it particularly has a minimum dimension (thickness) in a radial direction as will be explained in more detail below.

Surprisingly, the porous three-dimensional structure is capable of reducing or preventing tissue ingrowth. While concepts with porous structures were tested with the intention to promote tissue ingrowth in order to secure the position of the catheter within a blood vessel, it has been found out that the porous structure reduces or even prevents tissue ingrowth. More specifically, according to the invention it is desired that the porous three-dimensional structure promotes creation of an autologous graft (referred to as "autograft"), which forms a slippery surface and prevents overgrowth or ingrowth of tissue, in particular tissue of an inner wall of a blood vessel in which the catheter is located during operation of the blood pump. In other words, the patient's body creates its own coating over the catheter in the region of the porous structure, i.e. an autologous coating.

Thus, the basis of the autograft may be adsorption of proteins, in particular fibrinogen. Thus, more generally and in contrast to known catheters, which are designed to avoid tissue ingrowth, a catheter for an intravascular blood pump is provided, having on at least a portion of its outer surface a porous three-dimensional structure, which is able to adsorb proteins, e.g. fibrinogen and/or other proteins. After adsorption of fibrinogen, platelets and red blood cells may adhere to the fibrinogen layer. Further cells, e.g. myointimal cells and/or fibrinogen adhere to form a stable, homogenous autograft. Consequently, as already mentioned above, after formation of the autograft, which is an autologous coating, the autograft overlies at least the respective portion of the outer surface of the catheter which supports the porous three-dimensional structure, such that an outer surface of the autograft forms the outermost surface of the total assembly, comprising the catheter, the porous three-dimensional structure and the autograft.

The autograft is supported and held in place by the porous three-dimensional structure. It is slippery to allow the catheter to slide within the blood vessel without injuring or adhering to the inner vessel wall. The autograft formed on the porous structure reduces tissue irritation and thereby the natural reaction of the body to encapsulate foreign materials, and prevents the catheter from being overgrown by tissue such that the catheter can be easily removed even in long-term applications, e.g. of about three to six months, without causing trauma to the blood vessel. Even if trauma was caused in the blood vessel by initial mechanical irritation during insertion and advancement of the catheter, these injuries may heal with the catheter in place. The porous structure may be located along the entire length of the catheter or only on portions of the catheter body which are placed in larger vessels during operation of the blood pump to avoid mechanical irritation of the vessel or ingrowth.

In a preferred embodiment, the porous three-dimensional structure is formed by a sleeve arranged on the outer surface of the catheter body. The sleeve may comprise or may be formed of a textile material, such as a knitted, a knotted, a woven or a non-woven fabric or a combination thereof.

In particular, the sleeve may comprise a knitted fabric, preferably formed by warp knitting. Suitable warp knitting techniques may be used, such as 1x1 constructed knits (also known as tricot) or 2x1 constructed knits. Such warp knitted fabrics have good elasticity properties and are able to follow the movements and bends of the catheter without significantly affecting the mechanical characteristics of the catheter body. Furthermore, the mesh structure of a textile material, in particular a knitted fabric, provides a support structure with apertures and threads for adhesion of proteins and cells to form the aforementioned autograft.

The knitted fabric may include multi-filaments, each multi-filament preferably comprising 3 to 100 filaments, preferably 15 to 30 filaments, more preferably 24 filaments. The textile material of the sleeve may be produced by melt spinning process, which allows for creation of very thin filaments, e.g. having a diameter of about 1 µm to 100 µm, preferably 2 µm to 30 µm and more preferably 10 µm to 20 µm.

With regards to textile materials, e.g. for warp knitted fabrics, the linear mass density of fibers, yarns and thread is defined as the mass in grams per 1000 meters and measured in the unit "tex". One example of PET fabric with 24 filaments per multifilament may be PET 78dtex/24f. Other examples of suitable textile materials for the sleeve may be polyamide in the range of 17dtex/3f to 110dtex/34f, or PES in the range of 33dtex/24f to 180dtex/88f.

Further to the above-mentioned example, multi filaments with very thin filaments having a diameter of about e.g. 0.5 µm to 10 µm, preferably 1.7 µm to 5 µm and more preferably 2 µm to 4 µm may be used. Those filaments can be obtained by the so-called "island-in-the-sea"-process, for example. The number of filaments per thread has to be adapted according to the desired thread diameter.

The sleeve may have an elongate tubular body having a proximal end and a distal end. The sleeve may be attached to the tubular body of the catheter at least at the sleeve's proximal end and the sleeve's distal end. It will be appreciated that the sleeve may be attached to the catheter body also at further locations along the length of the sleeve's body or along the entire length of the sleeve. However, if the sleeve is secured only at its ends, the mechanical properties of the catheter substantially will not be affected. Advantageously, the sleeve is attached to the catheter body in a glue-free manner, i.e. without using an additional adhesive, which could fail during applications. For instance, the sleeve may be attached to the catheter body by solvent welding, where a solvent such as tetrahydrofuran (THF), dimethyl sulfoxide (DMSO), dimethylformamide (DMF) or the like is used to etch the catheter body while not affecting the sleeve material. The sleeve then firmly bonds with the catheter body.

The sleeve is advantageously tightly fitted on the outer surface of the tubular body. In other words, the sleeve may have an inner diameter substantially equal to or only slightly larger than the outer diameter of the catheter body. A tight fit of the sleeve over the catheter body may be preferable compared to a loose fit in order to avoid tissue or clots to accumulate inside the sleeve, i.e. between the sleeve and the catheter body. In case of a tight fit, the sleeve may be stiffer in a radial direction compared to an axial direction. In particular, the sleeve may be axially compliant, in particular with respect to axial extension and axial compression. More specifically, the sleeve may be axially extendable and axially compressible, which allows the sleeve to sit snuggly tight on the catheter while permitting the catheter to bend without causing wrinkles.

However, if desired, the sleeve may be fitted in a loose manner, i.e. may have an inner diameter larger than the outer diameter of the catheter body. If the sleeve is fitted in a loose manner such that a clearance may exist between the outer surface of the catheter body and an inner surface of the sleeve, the autograft may at least partially encapsulate the sleeve, i.e. the autograft may extend radially through the sleeve or from both a radially outer surface of the sleeve and the radially inner surface of the sleeve, and may thus contact the outer surface of the tubular body of the catheter.

Preferably, the porous structure comprises or is made of a non-absorbable material. The porous structure serves as a support structure or scaffold for the autograft. Thus, it is preferable if the porous structure is stable over time and during operation of the blood pump and does not resorb. Independent from its resorption characteristics, the porous structure may comprise a radiopaque material. This facilitates observation of the correct placement of the catheter, in particular correct placement of the porous structure. More specifically, it can be observed whether the porous structure, which may extend only along a portion of the length of the catheter, is placed correctly in critical areas of the blood vessels in which tissue overgrowth is most likely to occur. For instance, some of the threads of a textile material may comprise a radiopaque material to act as radiopaque markers.

Suitable materials for the porous structure may be polyethylene (PE), polypropylene (PP), polyamide (PA), polyether sulfone (PES), polyethylene terephthalate (PET), polyurethane (PU) and natural protein fiber, such as fiber of silk. The textile material of the sleeve may comprise filaments made of one or more of the aforementioned materials. The sleeve may be made of one material or a combination of materials. For instance, the warp and weft of a textile material may be made of different materials.

The porous structure may define a three-dimensional structure configured to promote adsorption of protein and cells. More specifically, the three-dimensional structure may include a plurality of apertures (or openings) and a plurality of webs (or struts) permitting formation of protein and cells in a radial inward direction. The apertures and webs may be formed by the apertures and threads of a textile material or other mesh or net structure. Alternatively, the porous three-dimensional structure may be formed from a foam or sponge-like structure or any other regular or irregular structure with pores which is able to promote adsorption of protein and cells. If desired, the porous structure may be integrally formed with the tubular body of the catheter on the outer surface thereof, or may be separately formed, e.g. in the form of the aforementioned sleeve, or may be manufactured directly onto the catheter, e.g. by electrospinning or spraying (as claimed).

The porous three-dimensional structure may comprise a single or more than one layer of one or more of the aforementioned configurations, such as a textile sleeve, mesh, foam or other porous structure. The individual layers may be formed of the same or different configurations. For instance, a foam-like or sponge-like structure may be combined with a textile sleeve surrounding the foam or sponge.

In an embodiment, the porous structure may define a plurality of first apertures and a plurality of second apertures, the first and second apertures being different in size. In other words, in case of a textile material the mesh size may vary. This may improve formation of the aforementioned autograft. With regards to a porous structure having more than one layer as described above, the aperture sizes may vary or may be the same in the individual layers to permit different cells to adhere in the different layers of the three-dimensional porous structure.

The porous structure, in particular the aforementioned three-dimensional structure, more specifically the aforementioned sleeve, may have a thickness of at least 20 µm, preferably at least 30 µm. The thickness of the porous three-dimensional structure is measured in a radial direction extending from the outer surface of the tubular body of the catheter to the outer surface of the porous structure. In case a clearance exists between the porous structure and the outer surface of the catheter body, the thickness of the porous structure may be measured from the inner surface of the porous structure to the outer surface of the porous structure in a radial direction. In other words, the wall thickness of the sleeve should have a minimum extension to improve the function of the sleeve as support structure for the autograft. In particular, a minimum radial thickness allows the autograft to grow in a radial inward direction from an outer surface of the porous structure such that the overall diameter substantially does not increase after initial formation of the autograft. It will be appreciated that the overall diameter of the catheter may be 12 F (4 mm) or less, preferably 9F (3 mm) or less, possibly 6F (2 mm) or 4F (1.3 mm), to avoid irritation or injury of the vessel wall. If the vessel size is too small relative to the implanted catheter, the caused mechanical irritation could again lead to overgrowth of tissue despite the presence of the autograft.

### BRIEF DESCRIPTION OF THE DRAWINGS

The foregoing summary, as well as the following detailed description of preferred embodiments, will be better understood when read in conjunction with the appended drawings. For the purpose of illustrating the present disclosure, reference is made to the drawings. The scope of the disclosure is not limited, however, to the specific embodiments disclosed in the drawings. In the drawings:
Figs. 1a and 1b schematically show an intravascular blood pump inserted into a patient's heart via different types of access.
Fig. 2 shows a cross-section of the catheter of the blood pump of Fig. 1 with the sleeve.
Fig. 3 shows an embodiment of a knit pattern for the sleeve.
Fig. 4 shows another embodiment of a knit pattern for the sleeve.
Fig. 5 shows an alternative embodiment for a porous three-dimensional structure.
Fig. 6 shows a further alternative embodiment for a porous three-dimensional structure.
Fig. 7 shows the catheter of Fig. 2 with a bi-layered porous three-dimensional structure.

### DETAILED DESCRIPTION

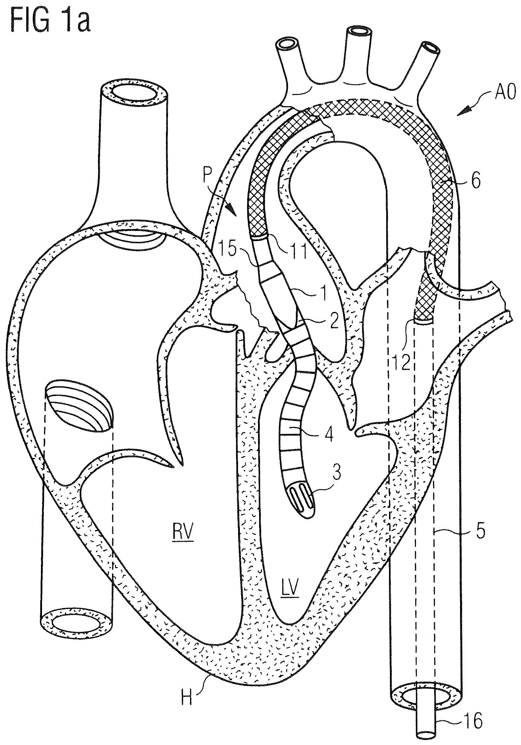

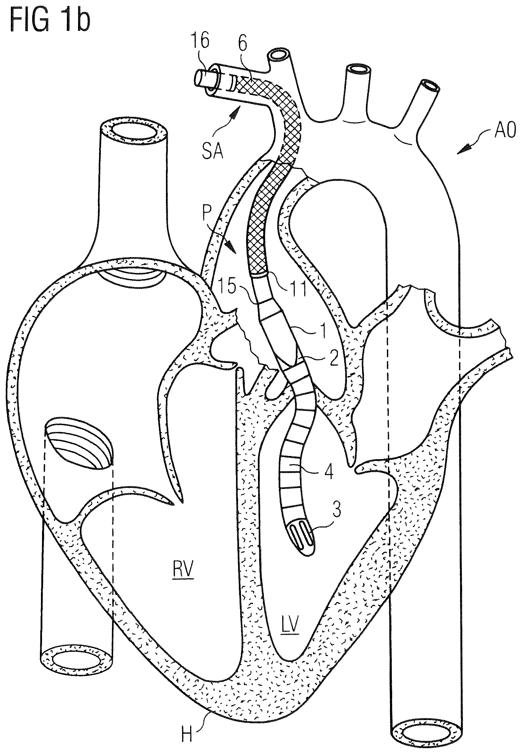

In Figs. 1a and 1b is illustrated an intravascular blood pump P inserted into a patient's heart H. More specifically, the blood pump P comprises a pumping device 1 attached to a catheter 5 by means of which the pumping device 1 is inserted into the left ventricle LV of the patient's heart H to pump blood from the left ventricle LV into the aorta AO. The shown application is only an exemplary application, and the blood pump P of the present invention is not limited to this application. For instance, reverse applications for the right ventricle RV may be envisioned. The blood pump P is percutaneously inserted e.g. via a femoral access and is advanced through the aorta AO into the heart H (see Fig. 1a). Alternatively, the blood pump P may be percutaneously inserted via an axillary access and advanced via a subclavian artery SA and through the aorta AO into the heart H (see Fig. 1b). The blood pump P is placed such that a blood flow outlet 2 is disposed outside the patient's heart H in the aorta AO, while a blood flow inlet 3 which is in flow communication with a flow cannula 4 is disposed inside the left ventricle LV. An impeller (not shown) is provided in the pumping device 1 to cause the blood flow from the blood flow inlet 3 to the blood flow outlet 2, and rotation of the impeller is caused by an electric motor (not shown) disposed in the pumping device 1.

The intravascular blood pump P is advanced into the patient's heart by means of the catheter 5, wherein the pumping device 1 is attached to a distal end 15 of the catheter 5 opposite a proximal end 16 of the catheter 5. As schematically illustrated in Fig. 1, the catheter 5 may contact the inner wall of the aorta AO, either during insertion or during operation or most likely both. This may cause irritation or injury of the vessel (triggered by the surface topology and a foreign material) wall and prompts overgrowth of the catheter 5 with bodily tissue. Proteins and other cells contained in the blood will adhere to the outer surface of the catheter 5. In particular, initially proteins and cells will adhere to the outer surface of the catheter 5. Therefore, known catheters tend to grow in the tissue of the vessel, which makes it difficult to remove the blood pump P from the patient's body. This will occur particularly in long-term application, where the blood pump P is in operation for several weeks or months. Removal of the ingrown catheter may cause injuries to the blood vessel.

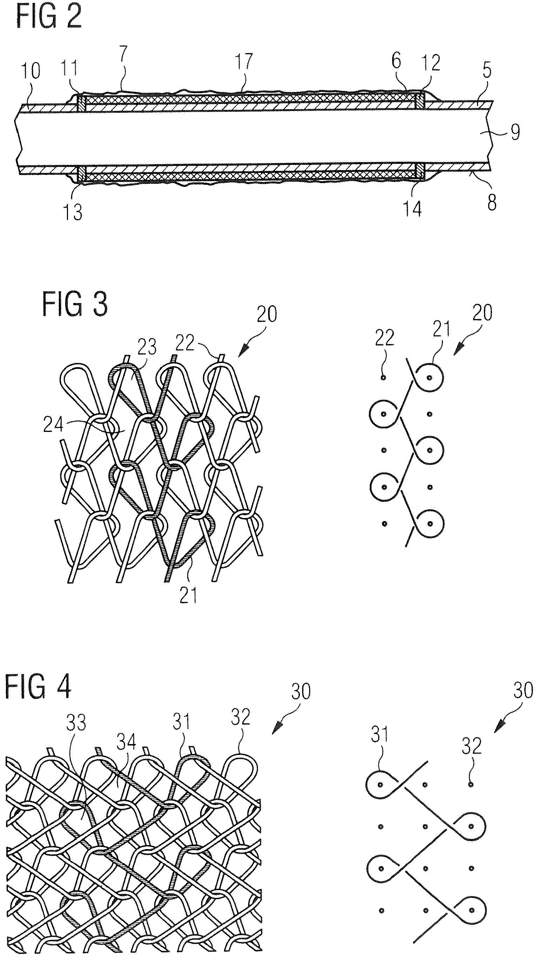

In order to reduce or avoid tissue ingrowth or overgrowth, a porous three-dimensional structure in the form of a textile sleeve 6 is provided along at least a portion of the catheter 5, in particular in a portion which tends to contact the inner vessel wall. Although the sleeve 6 induces adhesion of fibrinogen and could be considered to promote tissue ingrowth, it has been found that the opposite occurs due to formation of an autograft 7 (see Fig. 2) on the sleeve 6, which slides along the inner vessel wall as will be explained in more detail below.

Referring now to Fig. 2, a cross-section of the catheter 5 with the sleeve 6 mounted thereon is schematically illustrated. The catheter 5 has a tubular body 10 with an outer surface 8 and a lumen 9. The sleeve 6 has a tubular body 17 with a distal end 11 and a proximal end 12 and is attached to the body 10 of the catheter 5 by a suitable attachment technique, in particular solvent welding, which does not require the use of additional adhesive. As indicated in Fig. 2 at reference numerals 13 and 14, the sleeve 6 is attached to the catheter body 10 only at its distal end 11 and proximal end 12. Thus, the major part of the sleeve 6 between its ends 11, 12 is not attached to the outer surface 8 of the catheter body 10, such that the mechanical characteristics of the catheter 5 are substantially not affected by the sleeve 6. Further, it will be appreciated, while the sleeve 6 is shown to be formed of a single layer, it may comprise more than one layer. The layers may be identical or different, e.g. with respect to the size of the apertures formed by the knitted fabric described below and/or with respect to the layer materials.

The textile sleeve 6 may be made of a warp knitted fabric. A warp knitted fabric has good elasticity properties and provides a support structure with apertures to promote adsorption of the autograft 7. Other textile materials, such as knotted fabrics, woven fabrics, non-woven materials or a combination thereof may be used if they are suitable for inducing formation and for supporting the autograft. Examples of known warp knitted fabrics, which have been found to be particularly suitable for the sleeve 6, are schematically shown in Figs. 3 and 4. Fig. 3 shows a knitted fabric 20 as 1x1 constructed knits (also known as tricot). Fig. 4 shows a knitted fabric 30 as 2x1 constructed knits. In order to visualize the knitting patterns, a distinct thread 21 is marked in Fig. 3 among the threads 22. Apertures 23, 24 of different size are formed between the threads 21, 22 to promote adsorption of fibrinogen and further adhesion of blood cells to form the autograft 7. The same applies for the threads 31, 32 and the apertures 33, 34 of the knitted fabric 30 shown in Fig. 4. Preferably, the threads 21, 22, 31, 32 are formed by multi-filaments to enhance the three-dimensional structure of the sleeve 6.

The autograft 7 grows into the three-dimensional structure provided by the sleeve 6, in particular the warp knitted fabrics 20, 30 comprising multi-filaments as explained above. Since the autograft 7 is stably supported by the sleeve 6, it does not crumble or loosen but provides a slippery autologous coating which prevents adhesion of the catheter 5 to the inner vessel wall. The autograft 7 grows into the sleeve 6, such that the overall diameter of the catheter 5 substantially does not increase after initial formation of the autograft 7. The autograft 7 covers the sleeve 6 such that the catheter 5 will not be encapsulated in the region of the sleeve 6 as a foreign object and can be easily removed without causing trauma to the blood vessel. Furthermore, if trauma is caused initially upon insertion of the blood pump P into the patient, healing may start even during operation of the blood pump P with the catheter 5 in place in the blood vessel.

Thus, the intentional and desired adsorption of proteins and other cells on the sleeve 6 does not lead to ingrowth of the catheter 5 into the vessel wall, but has the unexpected effect that an autologous coating is formed which allows the catheter to slide freely inside the blood vessel and prevents ingrowth of the catheter 5. The unexpected effect can be described by the different dynamics. That means, while the formation of the autograft starts immediately, as soon as the porous structure is immersed in blood, the catheter overgrowth takes weeks. Thus, as soon as the autograft is present (typically within days), the stimulus for overgrowth from the vessel wall onto the adjacent catheter is stopped and overgrowth does not occur.

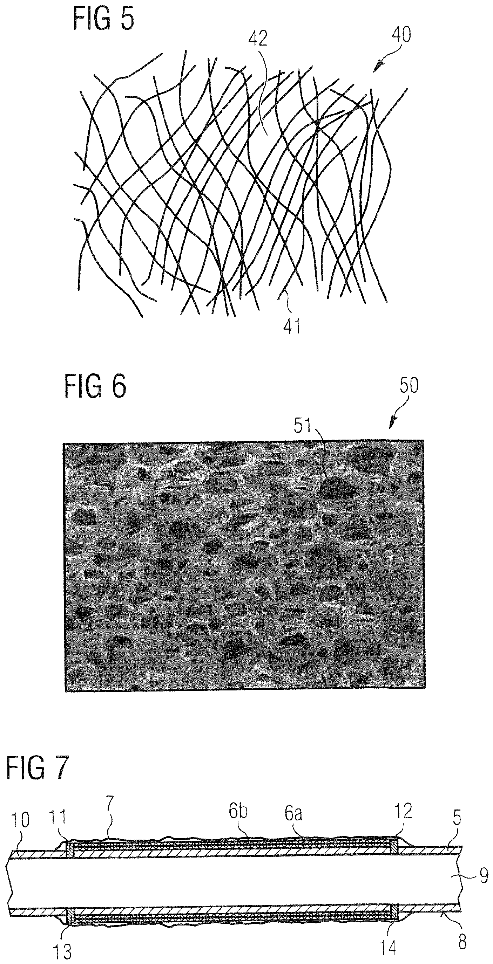

Figs. 5 and 6 schematically show further alternative embodiments for a porous three-dimensional structure which may be applied to a catheter instead of or possibly in addition to the aforementioned sleeve 6. Formation of the autograft occurs in the same way as explained above for the sleeve 6. Referring to Fig. 5, the porous three-dimensional structure 40 may be formed by electrospinning in one or more layers and may comprise a plurality of filaments 41 to form apertures 42 to permit formation of the autograft 7 as explained above. The filaments 41 and thus the apertures 42 may be arranged irregularly in desired dimensions. Electrospinning allows for manufacturing of the porous structure 40 directly onto the catheter 5. Likewise, a foam-like or sponge-like structure 50 having apertures 51 as shown in Fig. 6 may be formed directly on the catheter 5.

Fig. 7 shows the catheter 5 of Fig. 2 with a multi-layered three-dimensional structure on its outer surface 8. In this embodiment, the multi-layered structure has two porous layers 6a and 6b. The two layers 6a, 6b may be different. For instance, the inner layer 6a may be formed as a foam or by electrospinning of filaments directly on the outer surface 8 and the outer layer 6b may be formed as a textile sleeve.

## Claims

1. A catheter (5) of an intravascular blood pump (P) for percutaneous insertion into a patient's blood vessel, the catheter (5) having an elongate tubular body (10) which extends between a proximal end (16) and a distal end (15) of the catheter (5) and has an outer surface (8), the catheter (5) **characterised by** including a porous three-dimensional structure (6) on at least a portion of the outer surface (8) which is located in the patient's blood vessel during operation of the blood pump (P), wherein the porous three-dimensional structure (6) is directly formed onto the tubular body (10) of the catheter (5), preferably by electrospinning or spraying.

2. The catheter of claim 1, wherein the porous three-dimensional structure (6) is formed by a sleeve (6) arranged on the outer surface (8) of the tubular body (10).

3. The catheter of claim 2, wherein the sleeve (6) comprises at least one of the following: a textile material, a knitted fabric, a knotted fabric, a woven fabric and a nonwoven, wherein the knitted fabric (20; 30) is preferably formed by warp knitting, further preferably formed as 1x1 constructed knits (20) or 2x1 constructed knits (30), and/or wherein the knitted fabric (20; 30) preferably includes multi-filaments, each multi-filament preferably comprising 3 to 100 filaments, preferably 15 to 30 filaments, more preferably 24 filaments, wherein the multi filaments preferably have a diameter in a range of 0.5 µm to 10 µm, more preferably 1.7 µm to 5 µm and most preferably 2 µm to 4 µm.

4. The catheter of claim 2 or 3, wherein the sleeve (6) has an elongate tubular body (17) having a proximal end (12) and a distal end (11) and is attached to the tubular body (10) of the catheter (5) at least at the proximal end (12) and the distal end (11) of the sleeve (6), preferably in a glue-free manner, preferably only at the proximal end (12) and the distal end (11) of the sleeve (6).

5. The catheter of any one of claims 2 to 4, wherein the sleeve (6) is solvent-welded to the tubular body (10) of the catheter (5).

6. The catheter of any one of claims 2 to 5, wherein the sleeve (6) is tightly fitted on the outer surface (8) of the tubular body (10) of the catheter (5) or is loosely fitted on the outer surface (8) of the tubular body (10) of the catheter (5) such that a clearance exists between the tubular body (10) and an inner surface of the sleeve (6).

7. The catheter of any one of claims 2 to 6, wherein the sleeve (6) is stiffer in a radial direction as compared to an axial direction.

8. The catheter of any one of claims 1 to 7, wherein the porous three-dimensional structure (6) is formed from or comprises a foam or sponge-like structure.

9. The catheter of claim 1, wherein the porous three-dimensional structure (6) is integrally formed on an outer surface of the tubular body (10) of the catheter (5).

10. The catheter of any one of claims 1 to 3, wherein the porous three-dimensional structure (6) comprises melt-spun filaments, the melt-spun filaments preferably having a diameter in a range of 1 µm to 100 µm, more preferably 2 µm to 30 µm and most preferably 10 µm to 20 µm.

11. The catheter of any one of claims 1 to 10, wherein the porous three-dimensional structure (6) comprises a single layer (6) or comprises more than one layer (6a, 6b) which are preferably of different configuration, wherein the porous three-dimensional structure (6) preferably comprises a first layer (6a) having a foam-like or sponge-like structure and a second layer (6b) in the form of a textile sleeve, the second layer (6b) preferably surrounding the first layer (6a).

12. The catheter of any one of claims 1 to 11, wherein the porous three-dimensional structure (6) defines a plurality of first apertures and a plurality of second apertures, the first and second apertures being different in size.

13. The catheter of any one of claims 1 to 12, wherein the porous three-dimensional structure (6) comprises at least one of the following: a non-absorbable material, a radiopaque material, polyethylene, polypropylene, polyamide, polyether sulfone, polyethylene terephthalate, polyurethane and natural protein fibers, preferably silk fibers, and/or wherein the porous three-dimensional structure (6) is configured to promote adsorption of fibrinogen, the three-dimensional structure preferably including a plurality of apertures (23, 24; 33, 34) and a plurality of threads (21, 22; 31, 32) permitting adsorption of fibrinogen in a radial inward direction, and/or wherein the porous three-dimensional structure (6) has a thickness of at least 20 µm, preferably at least 30 µm.

14. The catheter (5) of any one of the preceding claims, wherein at least a portion of the outer surface (8) is configured to promote adsorption of proteins, preferably blood proteins, most preferably fibrinogen.

15. An intravascular blood pump (P) for percutaneous insertion into a patient's blood vessel, comprising a pumping device (1) and a catheter (5) of any one of claims 1 to 14.

## Patentansprüche

1. Katheter (5) einer intravaskulären Blutpumpe (P) zum perkutanen Einführen in ein Blutgefäß eines Patienten, wobei der Katheter (5) einen länglichen röhrenförmigen Körper (10) aufweist, der sich zwischen einem proximalen Ende (16) und einem distalen Ende (15) des Katheters (5) erstreckt und eine Außenfläche (8) hat, wobei der Katheter (5) eine poröse dreidimensionale Struktur (6) auf mindestens einem Teil der Außenfläche (8) aufweist, die sich während des Betriebs der Blutpumpe (P) in dem Blutgefäß des Patienten befindet, wobei die poröse dreidimensionale Struktur (6) direkt auf dem röhrenförmigen Körper (10) des Katheters (5) gebildet ist, vorzugsweise durch Elektrospinnen oder Sprühen.

2. Katheter nach Anspruch 1, wobei die poröse dreidimensionale Struktur (6) durch eine Hülse (6) gebildet wird, die auf der Außenfläche (8) des röhrenförmigen Körpers (10) angeordnet ist.

3. Katheter nach Anspruch 2, wobei die Hülse (6) mindestens eines der folgenden aufweist: ein textiles Material, ein Gewirk, ein Gestrick, ein Gewebe und einen Vliesstoff, wobei das Gewirk (20; 30) vorzugsweise durch Kettwirken gebildet ist, weiter vorzugsweise als 1x1-Konstruktion (20) oder 2x1-Konstruktion (30) gebildet ist, und/oder wobei das Gewirk (20; 30) vorzugsweise Multifilamente umfasst, wobei jedes Multifilament vorzugsweise 3 bis 100 Filamente, vorzugsweise 15 bis 30 Filamente, besonders bevorzugt 24 Filamente, umfasst, wobei die Multifilamente vorzugsweise einen Durchmesser in einem Bereich von 0.5 µm bis 10 µm, bevorzugter 1,7 µm bis 5 µm und am meisten bevorzugt 2 µm bis 4 µm aufweisen.

4. Katheter nach Anspruch 2 oder 3, wobei die Hülse (6) einen langgestreckten röhrenförmigen Körper (17) mit einem proximalen Ende (12) und einem distalen Ende (11) aufweist und zumindest am proximalen Ende (12) und am distalen Ende (11) der Hülse (6), vorzugsweise klebstofffrei, vorzugsweise nur am proximalen Ende (12) und am distalen Ende (11) der Hülse (6), am röhrenförmigen Körper (10) des Katheters (5) befestigt ist.

5. Katheter nach einem der Ansprüche 2 bis 4, wobei die Hülse (6) an den röhrenförmigen Körper (10) des Katheters (5) lösungsmittelgeschweißt ist.

6. Katheter nach einem der Ansprüche 2 bis 5, wobei die Hülse (6) fest auf der Außenfläche (8) des röhrenförmigen Körpers (10) des Katheters (5) sitzt oder lose auf der Außenfläche (8) des röhrenförmigen Körpers (10) des Katheters (5) sitzt, so dass ein Abstand zwischen dem röhrenförmigen Körper (10) und einer Innenfläche der Hülse (6) besteht.

7. der Katheter nach einem der Ansprüche 2 bis 6, wobei die Hülse (6) in radialer Richtung steifer ist als in axialer Richtung.

8. Katheter nach einem der Ansprüche 1 bis 7, wobei die poröse dreidimensionale Struktur (6) aus einer schaumstoff- oder schwammartigen Struktur gebildet ist oder diese umfasst.

9. Katheter nach Anspruch 1, wobei die poröse dreidimensionale Struktur (6) einstückig auf einer Außenfläche des röhrenförmigen Körpers (10) des Katheters (5) ausgebildet ist.

10. Katheter nach einem der Ansprüche 1 bis 3, wobei die poröse dreidimensionale Struktur (6) schmelzgesponnene Filamente umfasst, wobei die schmelzgesponnenen Filamente bevorzugt einen Durchmesser in einem Bereich von 1 µm bis 100 µm, noch bevorzugter 2 µm bis 30 µm und am meisten bevorzugt 10 µm bis 20 µm, aufweisen.

11. Katheter nach einem der Ansprüche 1 bis 10, wobei die poröse dreidimensionale Struktur (6) eine einzige Schicht (6) oder mehr als eine Schicht (6a, 6b) umfasst, die vorzugsweise unterschiedlich ausgebildet sind, wobei die poröse dreidimensionale Struktur (6) vorzugsweise eine erste Schicht (6a) mit einer schaumstoffartigen oder schwammartigen Struktur und eine zweite Schicht (6b) in Form einer textilen Hülle umfasst, wobei die zweite Schicht (6b) vorzugsweise die erste Schicht (6a) umgibt.

12. Katheter nach einem der Ansprüche 1 bis 11, wobei die poröse dreidimensionale Struktur (6) eine Vielzahl von ersten Öffnungen und eine Vielzahl von zweiten Öffnungen definiert, wobei die ersten und zweiten Öffnungen unterschiedlich groß sind.

13. Katheter nach einem der Ansprüche 1 bis 12, wobei die poröse dreidimensionale Struktur (6) mindestens eines der folgenden Materialien umfasst: ein nicht absorbierbares Material, ein röntgendichtes Material, Polyethylen, Polypropylen, Polyamid, Polyethersulfon, Polyethylenterephthalat, Polyurethan und natürliche Proteinfasern, vorzugsweise Seidenfasern, und/oder wobei die poröse dreidimensionale Struktur (6) so konfiguriert ist, dass sie die Adsorption von Fibrinogen fördert, wobei die dreidimensionale Struktur vorzugsweise eine Vielzahl von Öffnungen (23, 24; 33, 34) und eine Vielzahl von Fäden (21, 22; 31, 32) aufweist, die die Adsorption von Fibrinogen in einer radial nach innen gerichteten Richtung ermöglichen, und/oder wobei die poröse dreidimensionale Struktur (6) eine Dicke von mindestens 20 µm, vorzugsweise mindestens 30 µm, aufweist.

14. Katheter (5) nach einem der vorhergehenden Ansprüche, wobei mindestens ein Teil der Außenfläche (8) so konfiguriert ist, dass er die Adsorption von Proteinen, vorzugsweise Blutproteinen, am meisten bevorzugt Fibrinogen, fördert.

15. Intravaskuläre Blutpumpe (P) zur perkutanen Einführung in ein Blutgefäß eines Patienten, umfassend eine Pumpvorrichtung (1) und einen Katheter (5) nach einem der Ansprüche 1 bis 14.

## Revendications

1. Cathéter (5) d'une pompe sanguine intravasculaire (P) pour une introduction percutanée dans un vaisseau sanguin d'un patient, le cathéter (5) présentant un corps tubulaire oblong (10), lequel s'étend entre une extrémité proximale (16) et une extrémité distale (15) du cathéter (5) et présente une surface extérieure (8), le cathéter (5) étant **caractérisé en ce qu'**il inclut une structure tridimensionnelle poreuse (6) sur au moins une portion de la surface extérieure (8), laquelle se trouve dans un vaisseau sanguin du patient durant le fonctionnement de la pompe sanguine (P), dans lequel la structure tridimensionnelle poreuse (6) est directement formée sur le corps tubulaire (10) du cathéter (5), de préférence par électrofilage ou pulvérisation.

2. Cathéter selon la revendication 1, dans lequel la structure tridimensionnelle poreuse (6) est formée par un manchon (6) agencé sur la surface extérieure (8) du corps tubulaire (10).

3. Cathéter selon la revendication 2, dans lequel le manchon (6) comprend au moins un des éléments suivants : une matière textile, un tricot, un tissu noué, un tissu tissé, et un nontissé ; dans lequel le tricot (20; 30) est de préférence formé par tricotage chaîne, en outre et plus préférablement, est façonné sous la forme de tricots de construction 1 × 1 (20) ou de tricots de construction 2 x 1 (30), et/ou dans lequel le tricot (20; 30) inclut de préférence des multifilaments, chaque multifilament comprenant de préférence de 3 à 100 filaments, de préférence de 15 à 30 filaments, plus préférablement 24 filaments, dans lequel les multifilaments présentent de préférence un diamètre compris dans une plage allant de 0,5 µm à 10 µm, plus préférablement de 1,7 µm à 5 µm, et le plus préférablement de 2 µm à 4 µm.

4. Cathéter selon la revendication 2 ou 3, dans lequel le manchon (6) présente un corps tubulaire oblong (17) présentant une extrémité proximale (12) et une extrémité distale (11), et est fixé sur le corps tubulaire (10) du cathéter (5) au moins sur l'extrémité proximale (12) et l'extrémité distale (11) du manchon (6), de préférence d'une manière exempte de colle, de préférence uniquement sur l'extrémité proximale (12) et l'extrémité distale (11) du manchon (6).

5. Cathéter selon l'une quelconque des revendications 2 à 4, dans lequel le manchon (6) est soudé par solvant au corps tubulaire (10) du cathéter (5).

6. Cathéter selon l'une quelconque des revendications 2 à 5, dans lequel le manchon (6) est ajusté de manière serrée sur la surface extérieure (8) du corps tubulaire (10) du cathéter (5), ou est ajusté de manière lâche sur la surface extérieure (8) du corps tubulaire (10) du cathéter (5), de telle sorte qu'il existe un dégagement entre le corps tubulaire (10) et une surface intérieure du manchon (6).

7. Cathéter selon l'une quelconque des revendications 2 à 6, dans lequel le manchon (6) est plus raide dans une direction radiale, par comparaison avec une direction axiale.

8. Cathéter selon l'une quelconque des revendications 1 à 7, dans lequel la structure tridimensionnelle poreuse (6) est formée à partir d'une mousse ou d'une structure similaire à une éponge, ou comprend celle-ci.

9. Cathéter selon la revendication 1, dans lequel la structure tridimensionnelle poreuse (6) est formée d'un seul tenant sur une surface extérieure du corps tubulaire (10) du cathéter (5).

10. Cathéter selon l'une quelconque des revendications 1 à 3, dans lequel la structure tridimensionnelle poreuse (6) comprend des filaments filés par fusion, les filaments filés par fusion présentant de préférence un diamètre compris dans une plage allant de 1 µm à 100 µm, plus préférablement de 2 µm à 30 µm, et le plus préférablement de 10 µm à 20 µm.

11. Cathéter selon l'une quelconque des revendications 1 à 10, dans lequel la structure tridimensionnelle poreuse (6) comprend une couche unique (6), ou comprend plus d'une couche (6a, 6b), lesquelles sont de préférence de configuration différente ; dans lequel la structure tridimensionnelle poreuse (6) comprend de préférence une première couche (6a) présentant une structure similaire à une mousse ou similaire à une éponge, et une seconde couche (6b) sous la forme d'un manchon textile, la seconde couche (6b) entourant de préférence la première couche (6a).

12. Cathéter selon l'une quelconque des revendications 1 à 11, dans lequel la structure tridimensionnelle poreuse (6) définit une pluralité de premières ouvertures et une pluralité de secondes ouvertures, les premières et secondes ouvertures présentant des tailles différentes.

13. Cathéter selon l'une quelconque des revendications 1 à 12, dans lequel la structure tridimensionnelle poreuse (6) comprend au moins un des éléments suivants : une matière non absorbable, une matière radio-opaque, du polyéthylène, du polypropylène, du polyamide, de la polyéthersulfone, du polyéthylène téréphtalate, du polyuréthane, et des fibres de protéines naturelles, de préférence des fibres de soie, et/ou dans lequel la structure tridimensionnelle poreuse (6) est configurée pour favoriser l'adsorption de fibrinogène, la structure tridimensionnelle poreuse (6) incluant de préférence une pluralité d'ouvertures (23, 24; 33, 34) et une pluralité de filets (21, 22; 31, 32) permettant l'adsorption de fibrinogène dans une direction allant vers l'intérieur radiale, et/ou dans lequel la structure tridimensionnelle poreuse (6) présente une épaisseur d'au moins 20 µm, de préférence d'au moins 30 µm.

14. Cathéter (5) selon l'une quelconque des revendications précédentes, dans lequel au moins une portion de la surface extérieure (8) est configurée pour favoriser l'adsorption de protéines, de préférence de protéines du sang, le plus préférablement de fibrinogène.

15. Pompe sanguine intravasculaire (P) destinée à une introduction percutanée dans un vaisseau sanguin d'un patient, comprenant un dispositif de pompage (1) et un cathéter (5) selon l'une quelconque des revendications 1 à 14.
